# FASCICULE DE BREVET EUROPEEN

(11) **EP 2 005 918 B1**
(45) Date de publication et mention de la délivrance du brevet: **16.12.2009**
(21) Numéro de dépôt: 08104456.2
(22) Date de dépôt: 18.06.2008
(51) Int. Cl.: A61F 2/88, A61F 2/84

(54) **Prothèse tubulaire et nécessaire associé**
Röhrenförmige Prothese und entsprechendes Zubehör
Tubular prosthesis and associated kit

(30) Priorité: 20.06.2007 FR 0755892
(43) Date de publication de la demande: 24.12.2008
(73) Titulaire: Laboratoires Perouse, 60173 Ivry Le Temple (FR)
(72) Inventeur: Styrc, Witold, L-8190-GDL, Kopstal (LU)
(74) Mandataire: Jacobson, Claude

(56) Documents cités:
- WO-A-2005/070343
- WO-A-2006/047573
- FR-A- 2 865 926

## Description

La présente invention concerne une prothèse tubulaire déformable radialement, du type comprenant un corps tubulaire déformable entre un état rétracté de diamètre réduit et un état dilaté de diamètre plus grand, la prothèse comportant au moins deux crochets extérieurs délimitant entre eux une pince d'accrochage dans un tissu extérieur, les deux crochets étant portés par le corps et déplaçables entre une position écartée dans laquelle la pince est ouverte et une position rapprochée dans laquelle la pince est fermée.

Il est connu, pour différents types de traitement, de mettre en place une prothèse tubulaire à l'intérieur d'un vaisseau sanguin, qu'il s'agisse d'une veine ou d'une artère. De telles prothèses tubulaires sont généralement désignées par le terme "stent". WO-A-2005/079705 décrit une telle prothèse. La prothèse est amenée à l'intérieur du vaisseau alors qu'elle est dans son état rétracté, puis pour sa mise en place, la prothèse est dilatée pour s'appliquer contre la surface intérieure du vaisseau. Cette dilatation s'effectue soit automatiquement du fait de l'élasticité propre au treillis de la prothèse, soit sous l'action du gonflement d'un ballonnet intérieur, provoquant une déformation plastique du matériau constituant le treillis.

Pour éviter le déplacement ultérieur de la prothèse, l'extrémité du treillis métallique présente des paires de crochets formant chacune une pince d'accrochage pour immobiliser axialement la prothèse dans le vaisseau.

Chaque pince est ainsi délimitée par deux crochets portés par le treillis et déplaçables entre une position écartée dans laquelle la pince est ouverte et une position rapprochée dans laquelle la pince est fermée. Chaque crochet est filiforme et possède une extrémité fixée sur le treillis. L'autre extrémité du crochet est libre et forme une crosse d'accrochage dans le tissu délimitant le vaisseau sanguin.

Les deux extrémités de fixation de chaque pince sont portées par une même maille du treillis. Les crochets sont déplacés de leur position écartée vers leur position rapprochée par déplacement de leur extrémité de fixation lors de l'expansion de la maille portant la pince. La maille est d'abord maintenue rétractée lors de l'expansion de l'ensemble du treillis puis libérée pour la fermeture de la pince.

Toutefois, le treillis ne se déforme pas uniformément, notamment en fonction de la morphologie du vaisseau sanguin dans lequel il est implanté. Par suite, les extrémités libres des crochets formant une même pince peuvent s'écarter notablement l'une de l'autre après expansion du treillis. Ceci nuit à la solidité de la fixation du treillis dans le tissu délimitant le vaisseau.

Une prothèse selon le préambule de la revendication 1, est connue du document FR-A-2 865 926.

L'invention a pour but de proposer une prothèse tubulaire permettant un accrochage plus fiable du treillis de la prothèse tubulaire dans le vaisseau.

A cet effet, l'invention a pour objet une prothèse tubulaire du type précité, caractérisée en ce que la prothèse comprend un organe de guidage du déplacement d'au moins l'un des crochets lors de la déformation de la prothèse, l'organe de guidage délimitant un passage de guidage dans lequel est engagé au moins l'un des crochets.

Suivant des modes particuliers de réalisation, la prothèse tubulaire comporte l'une ou plusieurs des caractéristiques suivantes :
- les crochets comprennent un crochet de guidage et un crochet guidé, l'organe de guidage étant formé sur le crochet de guidage, le passage de guidage recevant le crochet guidé,
- l'organe de guidage est formé par une torsade du crochet de guidage sur lui-même,
- la torsade est réalisée sur au moins un tour,
- chaque crochet est lié au corps depuis une extrémité de liaison et les crochets d'une même pince sont mobiles l'un par rapport à l'autre lors de la déformation de la prothèse,
- le corps comporte un anneau élastique ceinturant le corps, l'anneau étant lié au corps et se déformant avec le corps entre un état rétracté et un état dilaté, l'anneau portant les deux crochets et les déplaçant entre leur position écartée et leur position rapprochée lors de sa déformation,
- le corps comprend un treillis déformable entre l'état rétracté et l'état dilaté et comportant des fils entrecroisés pour former un maillage de quadrilatères déformables, et en ce que chaque crochet est lié au treillis dans un coin du quadrilatère,
- chaque crochet est fixé au treillis à son extrémité de liaison,
- chaque crochet est prolongé à son extrémité de liaison par un brin torsadé autour du treillis,
- le corps tubulaire) comprend un tissu déformable entre l'état rétracté et l'état dilaté, les deux crochets étant fixés sur le tissu de telle sorte que le déploiement du tissu les déplace entre leur position écartée et leur position rapprochée.

L'invention est également relative à un nécessaire de traitement d'un vaisseau sanguin, caractérisé en ce qu'il comporte :
- une prothèse telle que décrite ci-dessus ;
- des moyens de retenue du treillis rétracté dans la région de la ou chaque pince ;
- un tube de largage du treillis délimitant un conduit de confinement de la prothèse dans son état rétracté.

Suivant un mode particulier de réalisation, le conduit de confinement du tube de largage comporte des canaux longitudinaux de réception des crochets.

L'invention sera mieux comprise à la lecture de la description qui va suivre, donnée uniquement à titre d'exemple et faite en se référant aux dessins, sur lesquels :
- la figure 1 est une vue en perspective d'une prothèse tubulaire à l'état dilaté ;
- la figure 2 est une vue en perspective de dessous à plus grande échelle de la prothèse à l'état dilaté dans une région comportant une pince dans une position ouverte ;
- la figure 3 est une vue analogue à la figure 2, la pince étant dans une position fermée ;
- la figure 4 est une vue en section transversale de la prothèse à l'état rétracté ;
- la figure 5 est une vue en perspective de la prothèse en cours d'implantation dans son état rétracté de la figure 4 ;
- la figure 6 est une vue en section à plus grande échelle de la prothèse dans son état rétracté dans une région comportant une pince en position ouverte ;
- la figure 7 est une vue analogue, à celle de la figure 4 de la prothèse dilatée avant accrochage ;
- la figure 8 est une vue en perspective de la prothèse dilatée avant accrochage ;
- la figure 9 est une vue analogue, à la figure 4 de la prothèse dilatée et accrochée après retrait des organes de retenue des pinces ;
- la figure 10 est une vue en perspective de la prothèse dilatée après retrait des organes de retenue des pinces ;
- la figure 11 est une vue en perspective et en élévation d'une variante de réalisation de la prothèse selon l'invention ;
- la figure 12 est une vue analogue à la figure 2 de la prothèse de la figure 11 ;
- la figure 13 est une vue analogue à la figure 3 de la prothèse de la figure 11 ;
- la figure 14 est une vue latérale en élévation d'une prothèse selon un troisième mode de réalisation de l'invention ; et
- la figure 15 est une vue en section d'une prothèse selon un quatrième mode de réalisation de l'invention.

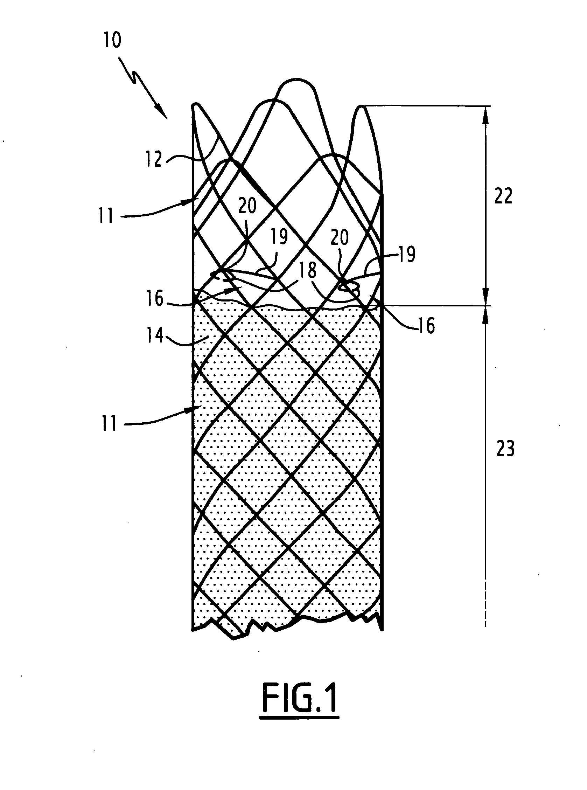

La prothèse tubulaire 10 représentée sur la figure 1 est destinée à être mise en place dans un vaisseau sanguin. Elle comporte un corps tubulaire 11 comprenant un treillis 12 noyé, sur l'essentiel de la longueur de la prothèse, dans un film 14 du corps 11. La prothèse comporte en outre, au voisinage de l'une de ses extrémités, trois pinces 16 régulièrement réparties angulairement à sa périphérie.

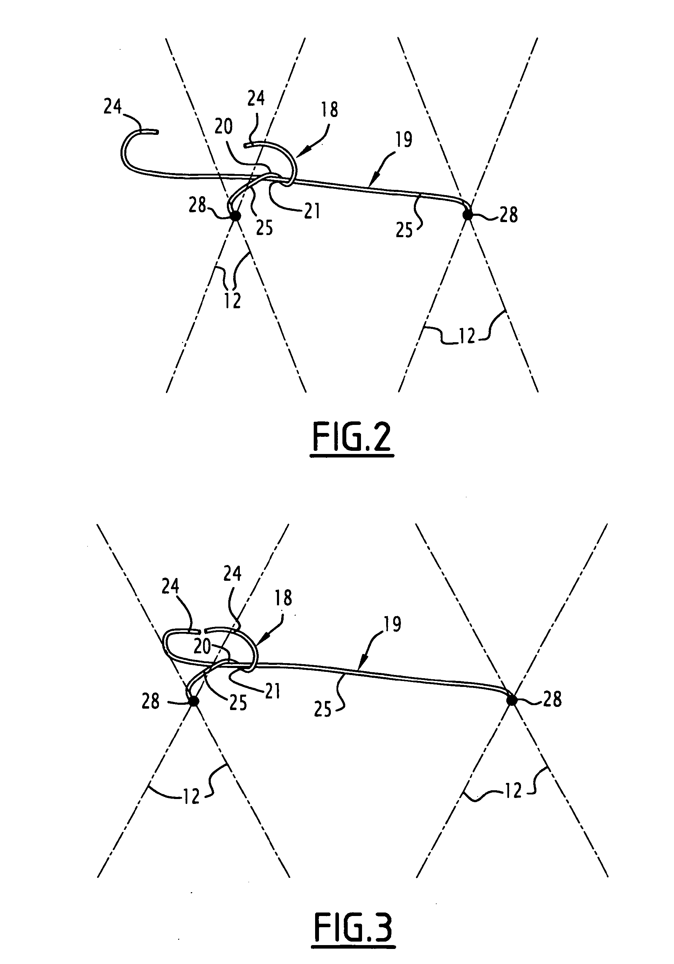

Chaque pince 16 est formée de deux crochets 18, 19 portés par le treillis métallique 12. Ces crochets 18, 19 sont déplaçables l'un par rapport à l'autre entre une position écartée dans laquelle la pince est ouverte et une position rapprochée dans laquelle la pince est fermée. La pince comprend en outre un organe 20 de guidage porté par l'un des crochets 18, 19 (voir figures 2 et 3). Le crochet 18, 19 portant l'organe 20 est un crochet 18 de guidage délimitant un passage 21 de guidage dans lequel est engagé l'autre des crochets 18, 19, qui est un crochet 19 guidé.

Dans l'exemple illustré, les pinces 16 sont prévues sur un tronçon d'extrémité 22 de la prothèse dépourvu de film 14, le treillis 12 n'étant ainsi pas recouvert dans cette région. En revanche, dans le tronçon principal noté 23 de la prothèse, le treillis 12 est noyé dans le film 14. En variante, les pinces 16 sont prévues sur tout le tronçon de la prothèse, notamment lorsque cette dernière est dépourvue de film.

Le treillis 12 est constitué d'acier inoxydable de qualité biocompatible. Il est réalisé par exemple par tressage ou tricotage d'un fil, déploiement axial d'un tube, ou par toute autre technique appropriée.

Dans le mode de réalisation illustré à la figure 1, le treillis 12 est constitué de deux faisceaux de fils enroulés en hélice en des sens opposés, les fils d'un même faisceau s'étendant généralement parallèlement les uns aux autres et transversalement aux fils du faisceau de fils enroulés en sens inverse.

Les fils des deux faisceaux d'enroulements opposés se croisent alternativement au-dessus et en dessous.

Le treillis 12 est de préférence élastiquement déformable par expansion radiale, entre un état rétracté de petit diamètre et un état dilaté de plus grand diamètre.

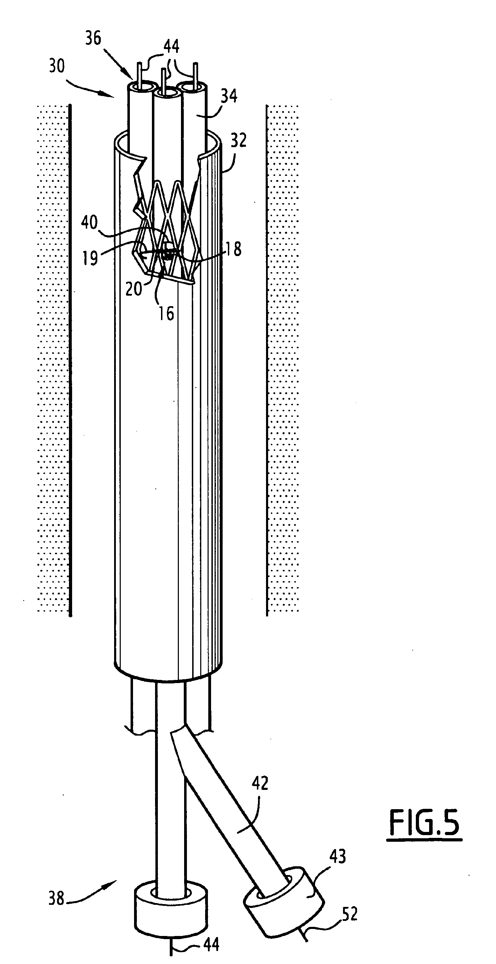

Dans son état dilaté, illustré sur la figure 1, les mailles du treillis forment des losanges généralement allongés dans le sens circonférentiel. Au contraire, et comme illustré sur la figure 5, à l'état rétracté de la prothèse, les mailles forment des losanges allongés parallèlement à l'axe de la prothèse.

En variante, la prothèse est plastiquement déformable, c'est-à-dire que le corps 11 et le treillis 12 possèdent une première forme stable de petit diamètre et une seconde forme stable de diamètre agrandi.

Sur le tronçon 23, le treillis 12 est entièrement noyé dans le film 14. Ce film est formé d'une matière extensible et étanche aux liquides qui emplit les mailles.

L'extensibilité de cette matière est suffisante pour que le film 14 suive la déformation du treillis 12 de son état rétracté à son état dilaté sans déchirure ni décollement, malgré la déformation des mailles et du treillis. Des matières appropriées sont un élastomère biocompatible qui peut être un caoutchouc naturel ou synthétique ou bien un polymère biocompatible tel qu'un polyuréthane.

L'enrobage du treillis 12 par le film 14 est obtenu par exemple par une technique de co-extrusion ou de trempage, après dégraissage du métal et son traitement par une substance primaire d'adhérence.

Comme illustré sur les figures 2 et 3, chaque crochet 18, 19 est formé d'un élément métallique filiforme dont une extrémité libre est recourbée vers l'extérieur pour former une crosse 24. Les crochets 18, 19 se chevauchent pour former la pince 16 entre les crosses 24 en regard. Les crosses 24 sont analogues.

Chaque crochet 18, 19 comprend un bras 25 prolongé par la crosse 24.

Le bras 25 du crochet guidé 19 constitue un tronçon rectiligne. En revanche, le bras 25 du crochet de guidage 18 comprend une boucle constituant l'organe de guidage 20. La boucle est réalisée par une torsade du crochet 18 sur lui-même sur un tour ou tout autre nombre de tours adapté. La torsade est réalisée autour du crochet guidé 19, ce qui facilite le montage des pinces 16. En outre, la torsade est réalisée par déformation plastique, de telle sorte qu'elle est permanente.

L'organe de guidage 20 formé par la torsade est situé au voisinage de la crosse 24 du crochet de guidage 18, dans le prolongement de la crosse 24

Le crochet guidé 19 est mobile suivant un axe de translation et deux axes orthogonaux de rotation dans le passage 21 délimité par l'organe 20. Les contraintes mécaniques pouvant s'exercer entre les deux crochets 18, 19 sont ainsi minimisées.

En variante, l'organe de guidage 20 est formé par torsade du crochet 18 sur lui-même sur plus d'un tour.

Dans une autre variante, le passage 21 n'est pas délimité par une boucle. Il est par exemple délimité par un anneau, ou un tube ou par tout autre élément de forme appropriée au guidage du crochet 19.

A son extrémité opposée à la crosse 24, chaque bras 25 est fixé au treillis métallique 12 dans les coins opposés d'une maille visibles sur les figures 2 et 3. Les crosses 24 font saillie vers l'extérieur par rapport au tronçon tubulaire délimité par le corps 11 et le treillis 12, et leurs extrémités recourbées s'étendent, au repos, dans un plan transversal à la prothèse tubulaire, c'est-à-dire perpendiculaire à son axe général.

Le diamètre des crosses 24 des crochets 18, 19 est compris entre 0,1 et plusieurs millimètres. De préférence, le diamètre des crosses 24 est inférieur au diamètre du passage 21.

La longueur du bras 25 du crochet guidé 19 est adaptée au diamètre de la prothèse 10. La longueur du bras 25 du crochet de guidage 18 est faible et inférieure à celle du bras 25 du crochet guidé 19 de telle sorte que l'organe 20 est très proche du point d'ancrage 28 du crochet 18. En effet, la faible longueur du bras 25 du crochet de guidage 18 assure la rigidité du bras 25 et accroît ainsi la fiabilité du guidage.

Le diamètre du passage 21 est supérieur au diamètre de la section du bras 25 du crochet 19.

Les longueurs des bras 25 sont telles que, dans l'état dilaté de la prothèse illustré sur la figure 1, les deux crosses 24 des crochets 18, 19 sont rapprochées et délimitent ensemble une boucle fermée ou pratiquement fermée.

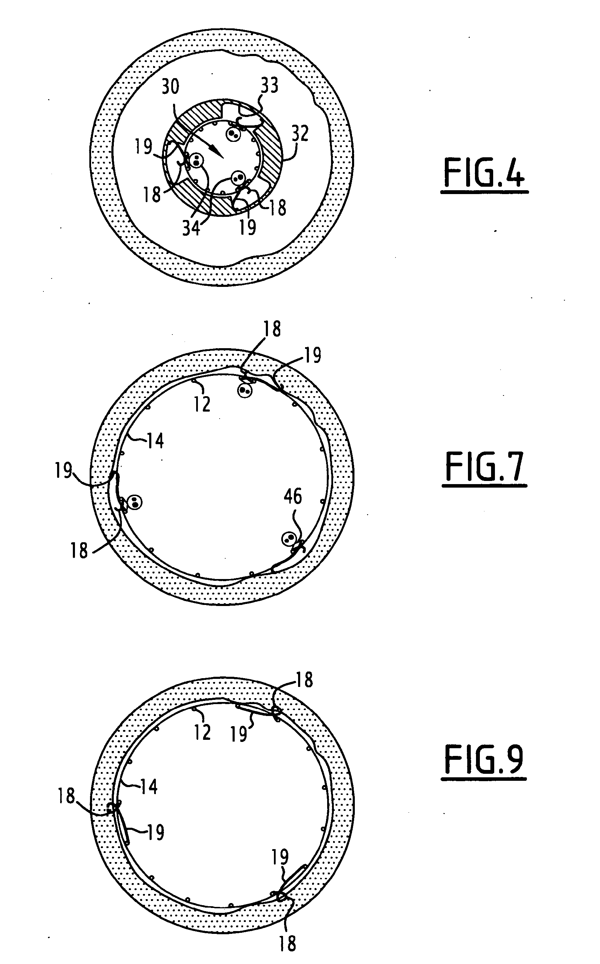

Initialement, et comme illustré sur les figures 4, 5, 6, 7 et 8, la prothèse est associée à des moyens 30 de retenue libérable des pinces 16 dans leur position ouverte.

En outre, la prothèse 10 dont les pinces 16 sont maintenues ouvertes est reçue, comme connu en soi, dans un tube de largage 32 à l'intérieur duquel la prothèse est confinée, dans son état rétracté.

Avantageusement, le conduit intérieur du tube 32 présente longitudinalement des canaux 33 de réception des extrémités des crochets 18, 19 faisant saillie par rapport à la surface généralement tubulaire du treillis métallique.

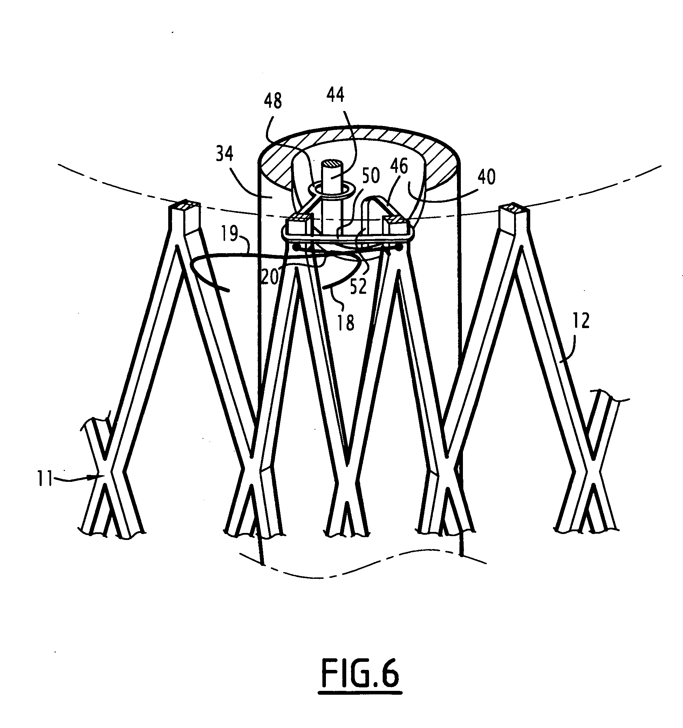

Comme illustré plus précisément sur les figures 5 et 6, chaque moyen de retenue d'une pince 16 ouverte comporte un tube flexible 34 formé par exemple en PEEK. Ce tube 34 s'étend longitudinalement entre une extrémité distale 36 destinée à être reçue dans le vaisseau sanguin et une extrémité proximale 38 destinée à être accessible par le chirurgien hors du corps du patient. Ainsi, le tube 34 a par exemple une longueur de un mètre.

Une ouverture de retenue 40 est ménagée latéralement dans le tube 34 généralement en regard de la pince 16 associée. Le tube 34 est équipé par ailleurs, au voisinage de son extrémité proximale 38, d'un embranchement latéral creux 42 équipé d'une bague 43 de blocage axial d'un fil coulissant.

Les moyens de retenue libérables 30 comprennent en outre une tige de retenue 44 engagée axialement dans le tube 34, et un fil de retenue 46 ceinturant la maille de la prothèse portant la pince 16.

La tige de retenue 44 s'étend d'un bout à l'autre du tube 34. Elle fait saillie hors du tube à l'extrémité proximale 38.

Cette tige est mobile dans le tube 34 entre une position de retenue dans laquelle la tige est en regard de l'ouverture 40 et une position de libération dans laquelle la tige 44 est à l'écart de l'ouverture 40 et est décalée vers l'extrémité proximale du tube 34.

Le fil de retenue 46 comprend un brin unique qui comporte à une extrémité un passant 48, une boucle de serrage 50 et un tronçon de commande 52 qui s'étend suivant toute la longueur du tube 34 de l'ouverture 40 à l'embranchement 42 hors duquel il fait saillie après avoir traversé la bague de blocage 43.

Le passant d'extrémité 48 est formé d'une boucle fermée de petit diamètre dans laquelle est engagée initialement la tige 44 lorsque celle-ci est dans sa position de retenue. La boucle de serrage 50 est formée par un tronçon du brin, engagée de manière coulissante au travers de deux mailles du treillis adjacentes à la maille portant la pince 16.

La boucle de serrage traverse l'ouverture 40 pour rejoindre le passant 48 à une extrémité et le tronçon de commande 52 à son autre extrémité. La longueur active de la boucle de serrage 50 est variable en fonction de la traction exercée sur le tronçon de commande 52, de sorte qu'elle contrôle la forme de la maille portant la pince 16, comme cela sera exposé dans la suite.

Initialement, avant mise en place, la prothèse est disposée dans le tube de largage 32 et les tronçons de commande 52 des moyens de retenue des pinces sont tendus, de sorte que les pinces sont maintenues ouvertes, comme illustré sur les figures 5 et 6. En effet, dans cette position, la boucle de serrage 50 resserre la maille portant la pince, de sorte que le losange définissant la maille est allongé suivant sa diagonale parallèle à l'axe de la prothèse.

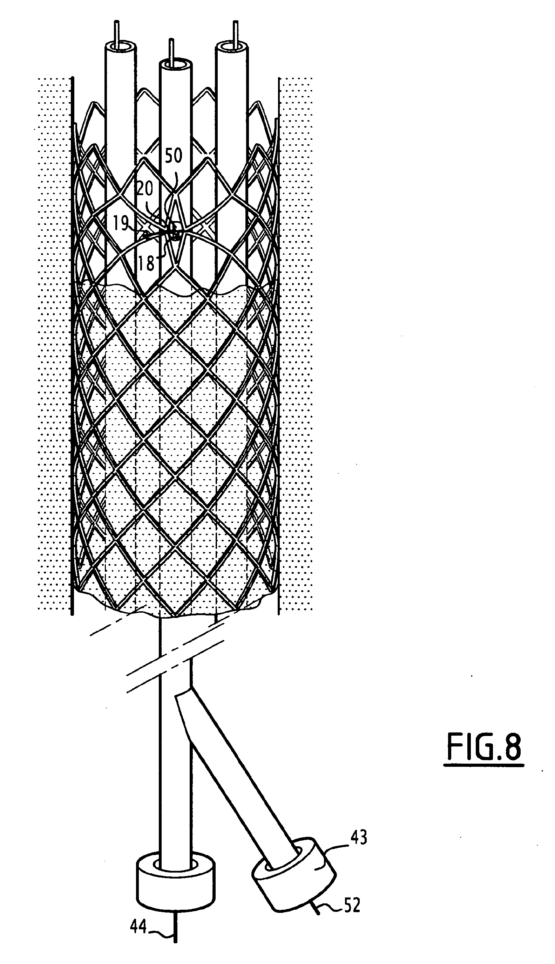

Pour la mise en place de la prothèse, celle-ci est introduite avec le tube 32 jusqu'à la zone de largage puis le tube 32 est retiré libérant ainsi la prothèse. Celle-ci se dilate et est alors plaquée contre la surface intérieure du vaisseau sanguin, comme illustré sur les figures 7 et 8.

Lors de cette dilatation, les mailles du treillis de la prothèse s'étendent grâce à l'élasticité du treillis suivant la diagonale périphérique de la prothèse permettant ainsi une augmentation du diamètre de la prothèse. En revanche, les mailles portant une pince restent contractées, comme illustré sur la figure 8 du fait de la boucle de serrage 50. Ainsi, les pinces 16 sont accostées contre la surface du vaisseau sanguin alors que celles-ci sont encore en position ouverte.

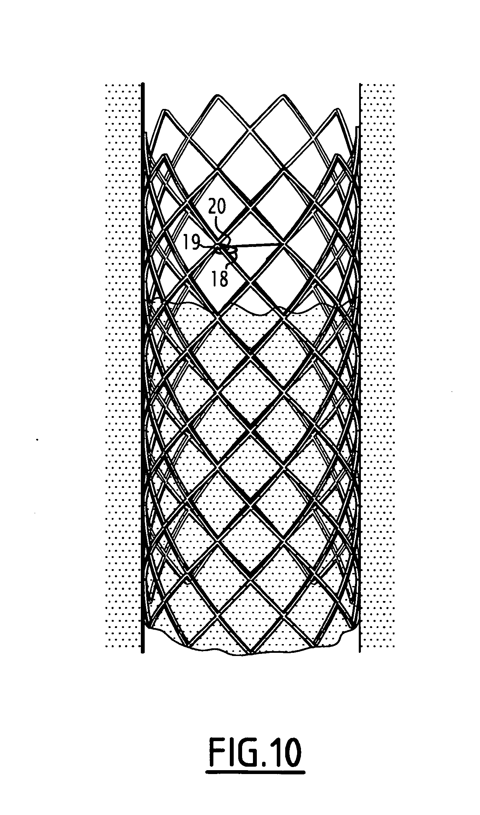

En agissant sur la bague de blocage 43, le praticien procède alors à la libération des fils de retenue afin d'autoriser la déformation élastique des mailles portant les pinces 16, et ainsi le rapprochement des deux crochets 18, 19 opposés, provoquant la fermeture de la pince 16 et la pénétration des crochets 18, 19 dans la paroi délimitant le vaisseau sanguin, comme illustré sur les figures 9 et 10.

Lors du rapprochement des deux crochets 18, 19, le crochet guidé 19 coulisse dans l'organe de guidage 20. L'organe de guidage 20 étant situé au voisinage de la crosse 24 du crochet de guidage 18, la crosse 24 du crochet guidé 19 se rapproche nécessairement de celle du crochet de guidage 18 lors de la fermeture de la pince 16. Cette fermeture est donc fiable quelque soit la déformation du treillis 12. Ainsi, même si la déformation de la maille portant la pince 16 n'est pas uniforme, la position relative des crosses 24 des deux crochets 18, 19 est sensiblement la même que lors d'une déformation uniforme de la maille. En effet, en position rapprochée, les crosses 24 sont maintenues au voisinage l'une de l'autre par l'organe de guidage 20 et la fonction de pince des crochets 18, 19 est préservée.

Après relâchement du fil de retenue 46, la tige 44 est amenée en position de libération, de sorte que le passant 48 est libéré de la tige 44. Le praticien tire alors sur le tronçon de commande 52 permettant que le fil de retenue 46 s'échappe du treillis métallique, en circulant au travers des deux mailles adjacentes à la maille portant la pince.

Ainsi, les moyens de retenue de la pince étant rendus indépendants de la prothèse, ceux-ci peuvent être extraits par voie endoluminale.

On comprend qu'une telle prothèse est retenue efficacement contre la surface interne du vaisseau par la présence des pinces qui sont maintenues élastiquement en position fermée sous l'action de la prothèse. De plus, les pinces étant refermées simultanément à la mise en place de la prothèse, l'installation d'une telle prothèse est relativement aisée.

En outre, le guidage du crochet 19 par le crochet 18 rigidifie la pince 16 formée par les deux crochets 18, 19. Si l'un des crochets 18, 19 est soumis à une contrainte externe, il est retenu par l'autre crochet 18, 19 par l'intermédiaire de l'organe 20 pour rester au voisinage l'un de l'autre. Si les crochets 18, 19 se déforment autour de leur point de fixation 28, leur position relative reste sensiblement inchangée et la fonction de la pince 16 préservée.

L'obtention de l'organe de guidage 20 au moyen de la torsade du crochet 18 sur lui-même assure un coût de fabrication faible. En outre, le crochet 19 est mobile dans le crochet 18 suivant deux axes orthogonaux de rotation et au moins un axe de translation. Les contraintes s'exerçant sur les crochets 18, 19 entre eux sont ainsi minimisées.

Dans une variante non représentée, l'organe de guidage est porté par le treillis. Les deux crochets sont alors engagés dans l'organe de guidage. L'organe de guidage est par exemple constitué d'une tige rigide soudée sur la maille portant la pince, la tige étant prolongée par un anneau formant l'organe de guidage.

En variante, l'organe de guidage 20 est de tout type connu adapté, comme par exemple un anneau solidaire du crochet 18.

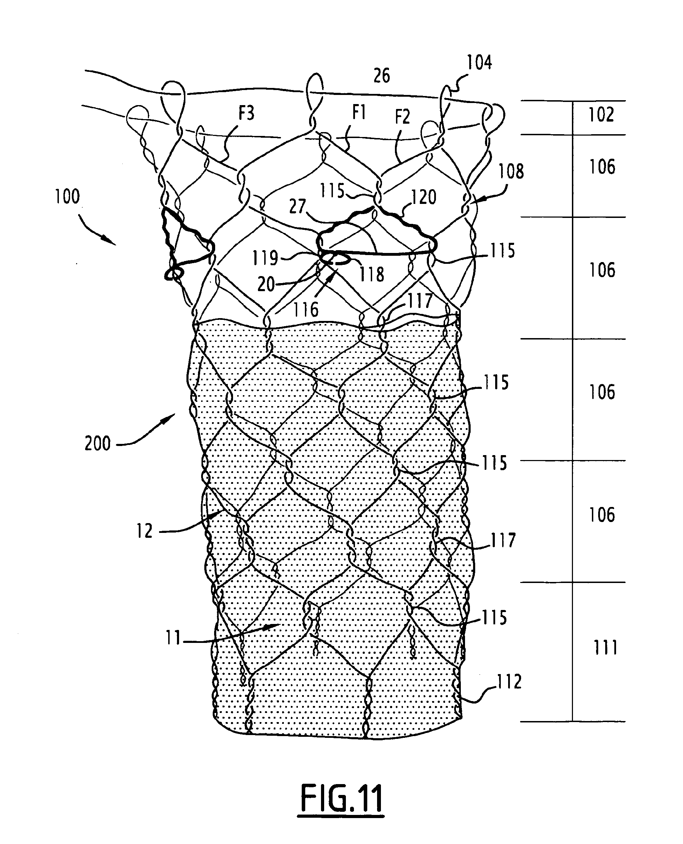

Sur la figure 11 est représentée un deuxième mode de réalisation d'une prothèse selon l'invention.

Le corps tubulaire 11 de la prothèse vasculaire 100 représentée sur la figure 11 comprend un treillis 12 lui-même formé par huit fils métalliques élastiques, tels que les fils F1, F2 et F3, torsadés ensemble d'une manière qui sera détaillée ci-dessous. Ces fils définissent, sur la longueur du treillis, plusieurs régions successives qui sont, de haut en bas sur la figure 11 :
- une région d'extrémité 102 à huit boucles 104 ;
- des régions successives 106 qui présentent chacune des couronnes périphériques de noeuds torsadés 108 ;
- une région d'extrémité 111 à torsades d'extrémité 112.

En considérant le fil F1 :
- la boucle 104 est constituée par la torsade du fil D1 sur lui-même, sur au moins un demi-tour ;
- chaque noeud 108 est constitué par la torsade du fil F1 avec un fil adjacent tel que F2, F3 sur un tour ou plus.

Ainsi, de part et d'autre de chaque noeud dit à torsade double et noté 115 où le fil est torsadé sur un nombre pair de demi-tours notamment égal à deux, chaque brin de fil qui forme le noeud s'étend suivant deux directions parallèles l'une à l'autre et proches l'une de l'autre.

Au contraire, en ce qui concerne les noeuds dits à torsade triple 117 où le fil est torsadé sur un nombre impair de demi-tours notamment égal à trois, chaque fil en part suivant deux directions qui forment entre elles un angle très inférieur à 180°, par exemple un angle droit ou un angle aigu comme représenté, pour constituer deux côtés adjacents d'une maille du treillis.

Comme indiqué sur la figure 11, dans l'exemple représenté, le fil F1 part d'une torsade 112 puis forme successivement un noeud 115, un noeud 117, un noeud 115, un autre noeud 115, un noeud 117, un noeud 115, un autre noeud 115, une boucle 104, un noeud 115, un autre noeud 115, un noeud 117, un noeud 115, un autre noeud 115, un noeud 117, un noeud 115 et une autre torsade 112.

Dans ce mode de réalisation, chaque pince notée 116 est formée d'un unique fil métallique 120 dont la partie courante est engagée et torsadée autour des fils délimitant le treillis, et dont les deux extrémités libres sont recourbées vers l'extérieur pour former des crochets 118, 119 analogues aux crochets 18, 19 du mode de réalisation précédent. Le crochet de guidage 118 comprend également un organe de guidage 20 délimitant un passage de guidage 21 dans lequel est engagé le crochet guidé 119.

Plus précisément et comme illustré sur les figures 11, 12 et 13, le fil 120 est torsadé depuis un noeud 115 autour de deux brins divergents angulairement décalés. Chaque branche du fil 120 est ensuite torsadée avec le noeud torsadé suivant et s'étend ensuite transversalement suivant un diamètre de la maille, pour former les deux bras 25 et les deux crosses 24 des crochets 118, 119.

Ainsi, comme dans le mode de réalisation précédent, la déformation de la maille portant la pince 116 provoque l'ouverture ou la fermeture de la pince 116, les deux crochets 118, 119 à extrémité recourbée se déplaçant l'un par rapport à l'autre.

L'organe de guidage 20 assure de la même manière la fiabilité de la fixation en maintenant les crochets 118, 119 en regard l'un de l'autre après déploiement de la prothèse 100.

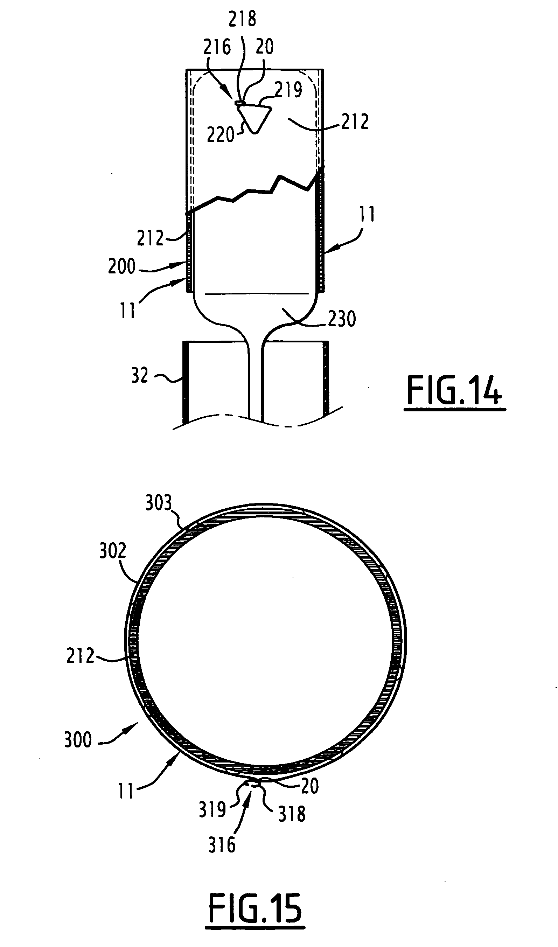

Dans un troisième mode de réalisation illustré sur la figure 14, le corps 11 de la prothèse vasculaire 200 est formé par un tube en tissu 212 déployable entre un état rétracté et un état dilaté de grand diamètre. Il s'agit, par exemple d'un tissu en dacron.

Deux crochets 218, 219 analogues aux crochets 118, 119 du deuxième mode de réalisation sont cousus sur le tube 212. Les crochets 218, 219 sont formés par un fil unique 220 en forme de triangle, les crochets 218, 219 et l'organe de guidage 20 étant placés au voisinage d'un sommet du triangle. Le déploiement du tissu 212 vers son état dilaté déplace les deux crochets 218, 219 entre leur position écartée et leur position rapprochée, ce déplacement étant guidé par l'organe de guidage 20.

Contrairement aux deux modes de réalisation précédents, le tissu 212 n'est pas déployable spontanément vers son état dilaté.

Initialement, la prothèse en tissu 200 est maintenue dans son état rétracté. Dans cet état, la pince 216 est ouverte et les crochets 218, 219 sont disposés à l'écart l'un de l'autre. La surface délimitée par le fil 220 en forme de triangle est alors minimale.

Le déploiement de la prothèse 200 est réalisé au moyen d'un ballonnet 230 gonflable entre un état rétracté et un état dilaté, inséré dans le tube en tissu 212. Le ballonnet 230 est amené, dans le tube 212 par le tube de largage 32 dans son état rétracté puis est gonflé.

Le déploiement du tube en tissu 212 provoque l'augmentation de la surface délimitée par le fil 220. Le crochet guidé 219 se rapproche alors du crochet de guidage 218 en étant maintenu proche de celui-ci par l'organe de guidage 20.

Les pinces 216 assurent, par leur accrochage radial dans le vaisseau sanguin, le maintien du tissu 212 dans son état dilaté. En outre, les deux crochets 218, 219 assurent comme dans les deux modes de réalisation précédents, l'accrochage axial de la prothèse 200.

Contrairement aux deux modes de réalisation précédents, le dispositif de largage ne possède pas de moyens de retenue 30 de la pince dans sa position ouverte. La fermeture de la pince 216 s'effectue au fur et à mesure du déploiement du tissu 212.

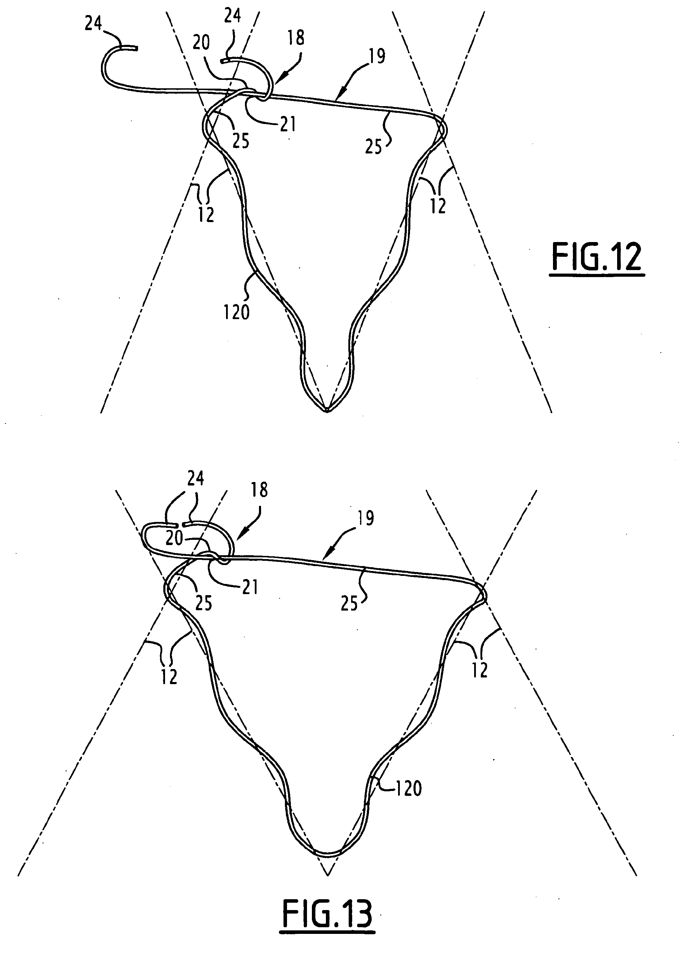

Dans un quatrième mode de réalisation illustré sur la figure 13, le corps 11 de la prothèse 300 est analogue à celui du troisième mode de réalisation. Cependant, la prothèse 300 comporte en outre un anneau déformable 302 ceinturant le tissu 212 et cousu avec ce dernier au moyen d'un fil 303. L'anneau 302 est ainsi prévu pour se déformer avec le tissu 212 entre un état rétracté dans lequel il présente un diamètre minimal et un état dilaté dans lequel il présente un diamètre maximal.

En outre, l'anneau 302 porte, à ses extrémités les deux crochets 318, 319, et l'organe de guidage 20 et les déplace entre leur position écartée et leur position rapprochée lors de sa déformation. Les deux crochets 318, 319 sont par exemple analogues aux crochets du premier mode de réalisation et ont leurs extrémités 28 soudées sur l'anneau 302.

En variante, l'anneau 302 est utilisé avec les prothèses 10, 100 des premier et deuxième modes de réalisation.

## Revendications

1. Prothèse tubulaire (10 ; 100 ; 200 ; 300) déformable radialement, du type comprenant un corps tubulaire (11) déformable entre un état rétracté de diamètre réduit et un état dilaté de diamètre plus grand, la prothèse (10 ; 100 ; 200 ; 300) comportant au moins deux crochets extérieurs (18, 19 ; 118, 119 ; 218, 219 ; 318, 319) délimitant entre eux une pince (16 ; 116 ; 216 ; 316) d'accrochage dans un tissu extérieur, les deux crochets (18, 19 ; 118, 119 ; 218, 219 ; 318, 319) étant portés par le corps (11) et déplaçables entre une position écartée dans laquelle la pince (16 ; 116 ; 216 ; 316) est ouverte et une position rapprochée dans laquelle la pince (16 ; 116 ; 216 ; 316) est fermée, **caractérisée en ce que** la prothèse (10 ; 100 ; 200 ; 300) comprend un organe (20) de guidage du déplacement d'au moins l'un des crochets (18, 19 ; 118, 119 ; 218, 219 ; 318, 319) lors de la déformation de la prothèse (10 ; 100 ; 200 ; 300), l'organe de guidage (20) délimitant un passage de guidage (21) dans lequel est engagé au moins l'un des crochets (18, 19 ; 118,119;218,219;318,319).

2. Prothèse (10 ; 100 ; 200 ; 300) selon la revendication 1, **caractérisée en ce que** les crochets (18, 19 ; 118, 119 ; 218, 219 ; 318, 319) comprennent un crochet (18 ; 118 ; 218 ; 318) de guidage et un crochet (19 ; 119 ; 219 ; 319) guidé, l'organe de guidage (20) étant formé sur le crochet (18 ; 118 ; 218 ; 318) de guidage, le passage (21) de guidage recevant le crochet guidé (19 ; 119 ; 219 ; 319).

3. Prothèse (10 ; 100 ; 200; 300) selon la revendication 1 ou 2, **caractérisée en ce que** les crochets (18, 19 ; 118,119 ; 218, 219 ; 318, 319) comprennent un crochet guidé (19 ; 119 ; 219 ; 319), le passage de guidage (21) recevant le croche guidé (19 ; 119 ; 219 ; 319), et **en ce que**, lors du rapprochement des deux crochets (18, 19 ; 118, 119 ; 218, 219 ; 318, 319), le crochet guidé (19 ; 119 ; 219 ; 319) coulisse dans l'organe de guidage (20).

4. Prothèse (10 ; 100 ; 200 ; 300) selon l'une quelconque des revendications précédentes, **caractérisée en ce que** l'organe de guidage (20) est formé par une torsade du crochet de guidage (18 ; 118 ; 218 ; 318) sur lui-même.

5. Prothèse (10 ; 100 ; 200 ; 300) selon la revendication 4, **caractérisée en ce que** la torsade est réalisée sur au moins un tour.

6. Prothèse (10 ; 100 ; 200 ; 300) selon l'une quelconque des revendications précédentes, **caractérisée en ce que** chaque crochet (18, 19 ; 118, 119 ; 218, 219 ; 318, 319) est lié au corps (11) depuis une extrémité de liaison et les crochets (18, 19 ; 118, 119 ; 218, 219 ; 318, 319) d'une même pince (16 ; 116 ; 216 ; 316) sont mobiles l'un par rapport à l'autre lors de la déformation de la prothèse (10 ; 100 ; 200 ; 300).

7. Prothèse (300) selon l'une quelconque des revendications précédentes, **caractérisée en ce que** le corps (11) comporte un anneau élastique (302) ceinturant le corps (11), l'anneau (302) étant lié au corps (11) et se déformant avec le corps (11) entre un état rétracté et un état dilaté, l'anneau (302) portant les deux crochets (318, 319) et les déplaçant entre leur position écartée et leur position rapprochée lors de sa déformation.

8. Prothèse (10 ; 100) selon l'une quelconque des revendications précédentes, **caractérisée en ce que** le corps (11) comprend un treillis (12) déformable entre l'état rétracté et l'état dilaté et comportant des fils entrecroisés pour former un maillage de quadrilatères déformables, et **en ce que** chaque crochet (18, 19; 118, 119) est lié au treillis (12) dans un coin du quadrilatère.

9. Prothèse (10) selon la revendication 8, **caractérisée en ce que** chaque crochet (18, 19) est fixé au treillis (12) à son extrémité de liaison.

10. Prothèse (10 ; 100) selon la revendication 8, **caractérisée en ce que** chaque crochet (118, 119) est prolongé à son extrémité de liaison par un brin torsadé (120) autour du treillis (12).

11. Prothèse (200) selon l'une quelconque des revendications 1 à 7, **caractérisée en ce que** le corps tubulaire (11) comprend un tissu (212) déformable entre l'état rétracté et l'état dilaté, les deux crochets (218, 219) étant fixés sur le tissu de telle sorte que le déploiement du tissu (212) les déplace entre leur position écartée et leur position rapprochée.

12. Nécessaire de traitement d'un vaisseau sanguin, **caractérisé en ce qu'**il comporte :
- une prothèse (10 ; 100 ; 300) selon l'une quelconque des revendications 1 à 11;
- des moyens (30) de retenue du corps (11) rétracté dans la région de la ou chaque pince (16) ;
- un tube (32) de largage du corps (11) délimitant un conduit de confinement de la prothèse (10) dans son état rétracté.

13. Nécessaire selon la revendication 12, **caractérisé en ce que** ledit conduit de confinement du tube de largage (32) comporte des canaux longitudinaux (33) de réception des crochets (18, 19 ; 118, 119 ; 318, 319).

## Claims

1. A radially deformable tubular prosthesis (10; 100; 200; 300), of the type comprising a tubular body (11) deformable between a smaller diameter collapsed state and a larger diameter expanded state, the prosthesis (10; 100; 200; 300) comprising at least two external hooks (18, 19; 118, 119; 218, 219; 318, 319) defining between them a clip (16; 116; 216; 316) for fixing in external tissue, the two hooks (18, 19 ; 118, 119 ; 218, 219 ; 318, 319) being borne by the body (11) and displaceable between a spaced-apart position in which the clip (16; 116; 216; 316) is open and a close-together position in which the clip (16; 116; 216; 316) is closed, **characterised in that** the prosthesis (10; 100; 200; 300) comprises a guiding member (20) for guiding displacement of at least one of the hooks (18, 19; 118, 119; 218, 219; 318, 319) during deformation of the prosthesis (10; 100; 200; 300), the guiding member (20) defining a guiding passage (21) in which at least one of the hooks (18, 19; 118, 119; 218, 219; 318, 319) is engaged.

2. A prosthesis (10; 100; 200; 300) according to claim 1, **characterised in that** the hooks (18, 19; 118, 119; 218, 219; 318, 319) comprise a guiding hook (18; 118; 218; 318) and a guided hook (19; 119; 219; 319), the guiding member (20) being formed on the guiding hook (18; 118; 218; 318), the guiding passage (21) receiving the guided hook (19; 119; 219; 319).

3. A prosthesis (10; 100; 200; 300) according to claim 1 or claim 2, **characterised in that** the hooks (18, 19; 118, 119; 218, 219; 318, 319) comprise a guided hook (19; 119; 219; 319), the guiding passage (21) receiving the guided hook (19; 119; 219; 319), and **in that**, when the two hooks (18, 19; 118, 119; 218, 219; 318, 319) are moved towards one another, the guided hook (19; 119; 219; 319) slides in the guiding member (20).

4. A prosthesis (10; 100; 200; 300) according to any one of the preceding claims, **characterised in that** the guiding member (20) is formed by twisting the guiding hook (18; 118; 218; 318) back on itself.

5. A prosthesis (10; 100; 200; 300) according to claim 4, **characterised in that** twisting is performed over at least one turn.

6. A prosthesis (10; 100; 200; 300) according to any one of the preceding claims, **characterised in that** each hook (18,19; 118, 119; 218, 219; 318, 319) is connected to the body (11) by a connecting end and the hooks (18, 19; 118, 119; 218, 219; 318, 319) of one and the same clip (16; 116; 216; 316) are movable relative to one another at the time of deformation of the prosthesis (10; 100; 200; 300).

7. A prosthesis (300) according to any one of the preceding claims, **characterised in that** the body (11) comprises a resilient ring (302) encircling the body (11), the ring (302) being connected to the body (11) and being deformed with the body (11) between a collapsed state and an expanded state, the ring (302) bearing the two hooks (318, 319) and moving them between their spaced-apart position and their close-together position at the time of its deformation.

8. A prosthesis (10; 100) according to any one of the preceding claims, **characterised in that** the body (11) comprises a lattice (12) deformable between the collapsed state and the expanded state and comprising wires intertwined to form a meshwork of deformable quadrilaterals, and **in that** each hook (18, 19; 118, 119) is connected to the lattice (12) at a corner of a quadrilateral.

9. A prosthesis (10) according to claim 8, **characterised in that** each hook (18,19) is fixed to the lattice (12) at its connecting end.

10. A prosthesis (10; 100) according to claim 8, **characterised in that** each hook (118, 119) is extended at its connecting end by a strand (120) twisted around the lattice (12).

11. A prosthesis (200) according to any one of claims 1 to 7, **characterised in that** the tubular body (11) comprises a fabric (212) deformable between the collapsed state and the expanded state, the two hooks (218, 219) being fixed to the fabric in such a way that unfolding of the fabric (212) moves them between their spaced-apart position and their close-together position.

12. A kit for treating a blood vessel, **characterised in that** it comprises:
- a prosthesis (10; 100; 300) according to any one of claims 1 to 11;
- holding means (30) for keeping the body (11) collapsed in the region of the or each clip (16);
- a tube (32) for releasing the body (11) which defines a duct confining the prosthesis (10) in its collapsed state.

13. A kit according to claim 12, **characterised in that** said confining duct defined by the releasing tube (32) comprises longitudinal channels (33) for accommodating the hooks (18, 19; 118, 119; 318, 319).

## Patentansprüche

1. Röhrenförmige Prothese (10, 100, 200, 300), die radial verformbar ist, des Typs der einen röhrenförmigen Körper (11) aufweist, der zwischen einem Zusammenziehungszustand mit verringertem Durchmesser und einem Dehnungszustand mit einem größeren Durchmesser verformbar ist, wobei die Prothese (10, 100, 200, 300) mindestens zwei äußere Haken (18, 19, 118, 119, 218, 219, 318, 319) aufweist, die zwischen einander eine Klemme (16, 116, 216, 316) zum Einhaken in ein äußeres Gewebe begrenzen, wobei die beiden Haken (18, 19, 118, 119, 218, 219, 318, 319) von dem Körper (11) getragen werden und zwischen einer Spreizposition, in der die Klemme (16, 116, 216, 316) geöffnet ist und einer Annäherungsposition, in der die Klemme (16, 116, 216, 316) geschlossen ist, bewegbar sind, **dadurch gekennzeichnet, dass** die Prothese (10, 100, 200, 300) ein Mittel (20) zum Führen der Bewegung von mindestens einem der Haken (18, 19, 118, 119, 218, 219, 318, 319) bei der Verformung der Prothese (10, 100, 200, 300) aufweist, wobei das Führungsmittel (20) einen Führungsdurchgang (21) begrenzt, in welchen mindestens einer der Haken (18, 19, 118, 119, 218, 219, 318, 319) eingreift.

2. Prothese (10, 100, 200, 300) gemäß Anspruch 1, **dadurch gekennzeichnet, dass** die Haken (18, 19, 118, 119, 218, 219, 318, 319) einen Führungshaken (18, 118, 218, 318) und einen geführten Haken (19, 119, 219, 319) aufweisen, wobei das Führungsmittel (20) an dem Führungshaken (18, 118, 218, 318) ausgebildet ist, wobei der Führungsdurchgang (21) den geführten Haken (19, 119, 219, 319) aufnimmt.

3. Prothese (10, 100, 200, 300) gemäß Anspruch 1 oder 2, **dadurch gekennzeichnet, dass** die Haken (18, 19, 118, 119, 218, 219, 318, 319) einen geführten Haken (19, 119, 219, 319) aufweisen, wobei der Führungsdurchgang (21) den geführten Haken (19, 119, 219, 319) aufnimmt, und **dadurch**, dass beim Annähern der beiden Haken (18, 19, 118, 119, 218, 219, 318, 319) der geführte Haken (19, 119, 219, 319) in dem Führungsmittel (20) gleitet.

4. Prothese (10, 100, 200, 300) gemäß einem der vorhergehenden Ansprüche, **dadurch gekennzeichnet, dass** das Führungsmittel (20) durch eine Verdrillung des Führungshakens (18, 118, 218, 318) mit sich selbst gebildet wird.

5. Prothese (10, 100, 200, 300) gemäß Anspruch 4, **dadurch gekennzeichnet, dass** die Verdrillung über mindestens eine Umdrehung realisiert ist.

6. Prothese (10, 100, 200, 300) gemäß einem der vorhergehenden Ansprüche, **dadurch gekennzeichnet, dass** jeder Haken (18, 19, 118, 119, 218, 219, 318, 319) von einem Verbindungsende aus mit dem Körper (11) verbunden ist, und die Haken (18, 19, 118, 119, 218, 219, 318, 319) einer selben Klemme (16, 116, 216, 316) bei der Verformung der Prothese (10, 100, 200, 300) relativ zueinander bewegbar sind.

7. Prothese (300) gemäß einem der vorhergehenden Ansprüche, **dadurch gekennzeichnet, dass** der Körper 811) einen elastischen Ring (302) aufweist, der den Körper (11) umfasst, wobei der Ring (302) mit dem Körper (11) verbunden ist und sich mit dem Körper (11) zwischen einem Zusammenziehungszustand und einem Dehnungszustand verformt, wobei der Ring (302) die beiden Haken (318, 319) trägt und sie bei seiner Verformung zwischen ihrer Spreizposition und ihrer Annäherungsposition bewegt.

8. Prothese (10, 100) gemäß einem der vorhergehenden Ansprüche, **dadurch gekennzeichnet, dass** der Körper (11) ein Gitter (12) aufweist, das zwischen dem Zusammenziehungszustand und dem Dehnungszustand verformbar ist und Fäden aufweist, die miteinander verflochten sind, um eine Vernetzung von verformbaren Vierecken zu bilden, und dass jeder Haken (18, 19, 118, 119) in einer Ecke des Vierecks mit dem Gitter (12) verbunden ist.

9. Prothese (10) gemäß Anspruch 8, **dadurch gekennzeichnet, dass** jeder Haken (18, 19) an seinem Verbindungsende an dem Gitter (12) befestigt ist.

10. Prothese (10, 100) gemäß Anspruch 8, **dadurch gekennzeichnet, dass** jeder Haken (118, 119) an seinem Verbindungsende durch eine verdrillte Faser (120) um das Gitter (12) herum verlängert ist.

11. Prothese (200) gemäß einem der Ansprüche 1 bis 7, **dadurch gekennzeichnet, dass** der röhrenförmige Körper (11) ein Gewebe (212) aufweist, das zwischen dem Zusammenziehungszustand und dem Dehnungszustand verformbar ist, wobei die beiden Haken (218, 219) derart an dem Gewebe befestigt sind, dass sie das Ausdehnen des Gewebes (212) zwischen ihrer Spreizposition und ihrer Annäherungsposition bewegt.

12. Satz zur Behandlung eines Blutgefäßes, **dadurch gekennzeichnet, dass** er aufweist:
- eine Prothese (10, 100, 300) gemäß einem der Ansprüche 1 bis 11,
- Mittel (30) zum Halten des zusammengezogenen Körpers (11) in dem Bereich der oder jeder Klemme (16),
- ein Rohr (32) zum Erweitern des Körpers (11), das eine Röhre zum Umschließen der Prothese (10) in ihrem Zusammenziehungszustand begrenzt.

13. Satz gemäß Anspruch 12, **dadurch gekennzeichnet, dass** die Umschließungsröhre des Erweiterungsrohrs (32) längsverlaufende Kanäle (33) zum Aufnehmen der Haken (18, 19, 118, 119, 318, 319) aufweist.
